# FASCICULE DE BREVET EUROPEEN

(11) **EP 0 519 807 B1**
(45) Date de publication et mention de la délivrance du brevet: **09.08.1995**
(21) Numéro de dépôt: 92401673.6
(22) Date de dépôt: 16.06.1992
(51) Int. Cl.: C07C 25/24, C07C 17/25, C07B 35/06

(54) **Procédé pour deshydrochlorer les 1,1-bis(r-phényl)-2,2,2-trichloroéthanes**
Verfahren zur Dehydrochlorierung von 1,1-bis(R-Phenyl)-2,2,2-trichloroethan
Process for the dehydrochlorination of 1,1-bis(R-phényl)-2,2,2-trichloroethanes

(30) Priorité: 19.06.1991 FR 9107522
(43) Date de publication de la demande: 23.12.1992
(73) Titulaire: ELF ATOCHEM S.A., 92800 Puteaux (FR)
(72) Inventeur: Castella Sola, Jaume, E-08911 Badalona (ES); Palencia Adrubau, Jaime, E-08911 Badalona (ES)

(56) Documents cités:
- PL-A- 110 642
- CHEMICAL ABSTRACTS, vol. 96, no. 15, 12 Avril 1982, Columbus, Ohio, US;abstract no. 122386H, page 659 ;

## Description

La présente invention concerne un procédé pour deshydrochlorer les 1,1-bis(R-phényl)-2,2,2-trichloroéthanes. La famille des 1,1-bis(R-phényl)-2,2,2-trichloroéthanes est connue. Par exemple : le 1,1-bis(chlorophényl)-2,2,2-trichloroéthane (DDT) est un produit intermédiaire de la synthèse du 1,1-bis(chlorophényl)-2,2,2-trichloroéthanol communément appelé dicofol, acaricide utilisé pour le coton et les arbres fruitiers. Le DDT est déshydrochloré en 1,1-bis(chlorophényl)-2,2-dichloroéthylène (DDE) qu'on chlore pour obtenir le 1,1-bis (chlorophényl)-1,2,2,2-tétrachloroéthane (chloroDDT) qu'on hydrolyse en dicofol. 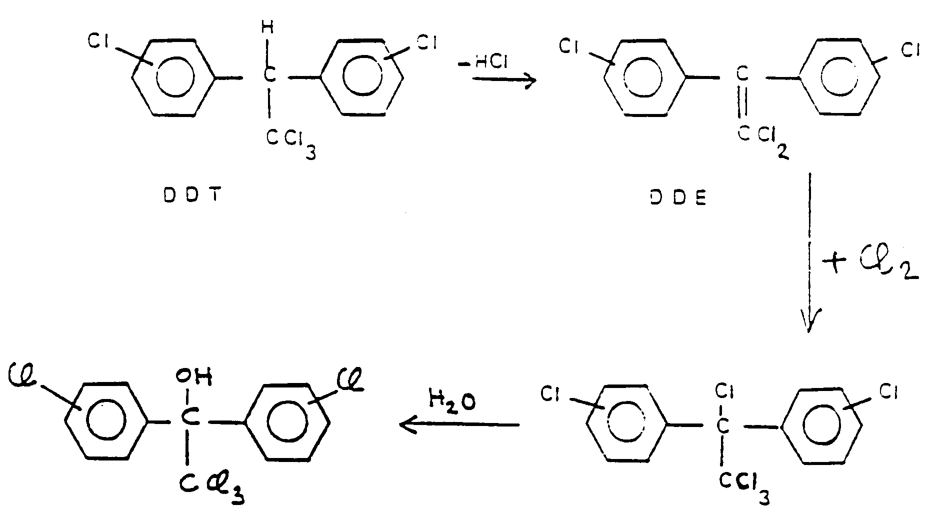
Le brevet polonais PL 110642 (Chemical Abstracts 1982, Vol. 96 : 122386 h) décrit la déshydrochloration du diphényltrichloroéthane (C₆H₅)₂CH-CCl₃ en diphényl-dichloroéthylène (C₆H₅)₂C=CCl₂ en opérant dans le benzène avec de la soude et du chlorure de méthylbis(polyoxyéthylène) stearyl ammonium.

Le brevet russe SU 899524 (Chemical Abstracts 1982, Vol. 97 : 55461 j) décrit la déshydrochloration du 1,1-bis(p-chlorophényl)-2,2,2-trichloroéthane (p - Cl C₆ H₄)₂ CH - CCl₃ par un hydroxyde alcalin en opérant dans l'éthanol ou l'isopropanol.

Le brevet polonais PL 112229 (Chemical Abstracts 1982, Vol. 96 : 162 301 h) décrit la déshydrochloration du bis (parahydroxyphényl) trichloroéthane, (4 - HO C₆ H₄)₂ CH - CCl₃ dans le méthanol en présence d'une résine basique d'un ammonium quaternaire et échangeuse d'ions.

La présence d'un solvant tel que le benzène, l'éthanol, l'isopropanol ou le méthanol peut être gênante quand on doit, aprés l'opération de déshydrochloration, procéder à une chloration, car ce solvant peut se chlorer.

Si on doit isoler le produit obtenu à la déshydrochloration, la présence de solvant rend cette séparation plus compliquée.

La demanderesse a découvert qu'il n'était pas nécessaire d'utiliser un solvant.

La présente invention est donc un procédé de déshydrochloration des 1,1-bis(R-phényl)-2,2,2-trichloroéthane à l'aide d'une solution aqueuse d'un hydroxyde alcalin caractérisé en ce qu'on opère en l'absence de solvant et en présence d'un agent de transfert de phase.

Le radical R peut représenter un ou plusieurs halogènes, alkyls, groupes hydroxy (OH), groupes NO₂, alkoxy. Parmi les alkyls, on peut citer le méthyl, l'éthyle, le propyle ou l'isopropyle. Parmi les groupes alkoxy on peut citer le méthoxy et l'éthoxy. On peut aussi avoir une combinaison de plusieurs substituants sur le même phényle, des substituants différents sur chacun des phényles seulement un phényl substitué ou toute combinaison de ces substituants. Bien qu'on puisse opérer dans une large plage de pression, on se place à la pression suffisante pour maintenir le 1,1-bis(R-phényl)-2,2,2-trichloroéthane et la solution aqueuse de l'hydroxyde alcalin à l'état liquide. L'agent de transfert de phase est un produit permettant le contact entre le produit à deshydrochlorer et l'hydroxyde alcalin. Il existe de nombreux produits ayant cette fonction. On peut à titre d'exemple utiliser les ammonium quaternaires. On a utilisé avec succès le chlorure de diméthyl lauryl benzyl ammonium.

On ne sortirait pas du cadre de l'invention en ajoutant la solution d'hydroxyde alcalin en plusieurs fois au cours de la réaction, ou en complétant la teneur en hydroxyde alcalin du milieu réactionnel par des additions d'hydroxyde alcalin anhydre ou très concentrés tel que de la potasse ou de la soude en écailles ceci permettant la réduction de la consommation d'hydroxyde alcalin, tout en maintenant en solution le chlorure de sodium formé. En fin de réaction, il suffit de séparer la phase organique contenant le produit à deshydrochlorer et le produit deshydrochloré et la phase aqueuse alcaline ; on peut opérer par simple décantation.

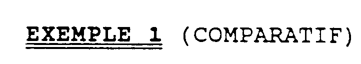

On fond 420 g de 1,1-bis(chlorophényl)-2,2,2-trichloroéthane et on coule, avec agitation et à une température de 100-105°C, 270 g d'hydroxyde de soude 50 %. Après 20 h de réaction on dilue avec 300 g d'eau. La phase organique décantée est lavée deux fois avec 100 g d'eau et on obtient 371.9 g d'un mélange de 1,1-bis(chlorophényl)-2,2-dichloroéthylène et 1,1-bis(chlorophényl)-2,2,2-trichloroéthane (15,5 %).

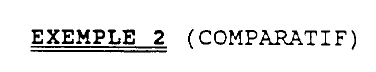

A une solution de 250 g de 1,1-bis(chlorophényl)-2,2,2-trichloroéthane en 250 g d'isobutanol, on ajoute 60,6 g d'hydroxyde de soude 50 % et deux gouttes d'un agent antimousse. On chauffe avec agitation jusqu'à distillation de l'azéotrope isobutanol-eau en maintenant a reflux pendant 1 h. On sépare l'eau par distillation azéotropique de l'eau-isobutanol tout en ajoutant de l'isobutanol.

Après séparation de l'eau on filtre, à température ambiante, la phase solide séparée et on distille l'isobutanol de la phase liquide. On obtient 217,9 g de 1,1-bis(chlorophényl)-2,2-dichloroéthylène (r=97,2 %) avec 0,18 % de 1,1-bis(chlorophényl)-2,2,2-trichloroéthane.

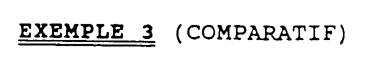

A une solution de 250 g de 1,1-bis(chlorophényl)2,2,2-trichloroéthane en 100 g de monochlorobenzène, à 50°C, on ajoute 201,5 g d'hydroxyde de soude 28 % et 2,0 g de chlorure de diméthyl lauryl benzylammonium. On chauffe à reflux (90°C) pendant 20 h. On sépare par décantation la phase organique qu'on lave à l'eau trois fois 100 g. Après distillation du monochlorobenzène, on obtient 219,3 g de 1,1-bis(chlorophényl)-2,2-dichloroéthylène (r=97,8 %) avec 0,10 % de 1,1-bis(chlorophényl)-2,2,2-trichloroéthane.

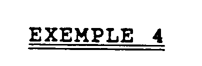

Sur 248,8 g d'une solution aqueuse d'hydroxyde de soude 27,2 % maintenue à 93°C, on additionne 150 g du 1,1-bis(chlorophényl)-2,2,2-trichloroéthane solide. On ajoute 1,4 g de chlorure de diméthyl lauryl benzyl ammonium. La température augmente de 93 à 103°C. On continue l'addition du 1,1-bis(chlorophényl)-2,2,2-trichloroéthane solide jusqu'à un total de 300 g. On ajoute à nouveau 1,1 g de chlorure de diméthyl lauryl benzyl ammonium et on maintient la température entre 100 et 105°C pendant 14 h 30 mn. Par décantation, on sépare et lave la phase organique avec trois fois 100 g d'une solution d'acide sulfurique 1 N et de l'eau (100 g). On obtient 263,7 g de 1,1-bis(chlorophényl)-2,2-dichloroéthylène (r=98,0 %) avec 0,09 % de 1,1-bis(chlorophényl)-2,2,2-trichloroéthane.

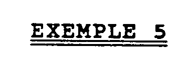

Sur la phase aqueuse basique d'une opération précédente de déshydrochloration, on charge à 100°C 990 kg de 1,1-bis(chlorophényl)-2,2,2-trichloroéthane solide. La durée de la charge est de 2 h afin d'éviter la formation de masses solides à l'intérieur du réacteur. On ajoute 5 kg de chlorure de diméthyl benzyl lauryl ammonium tout en maintenant en agitation intense pendant 4 h à 100°C. Après 1 h de repos, on sépare la phase aqueuse et on ajoute 268 kg d'hydroxyde de soude 50 % et on continue la déshydrochloration avec agitation à 100°C et 10 h. Après dilution avec 560 kg d'eau et 1 h de repos, on soutire la phase organique et on retient la phase aqueuse pour l'opération suivante. La phase organique est ensuite lavée avec 3 fois 250 kg d'une solution d'acide sulfurique 1 N et on obtient 879 kg de 1,1-bis(chlorophényl)-2,2-dichloroéthyléne.

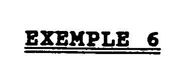

On opère comme dans l'exemple 5, sauf qu'au départ on dispose de 1,1-bis(chlorophényl)-2,2,2-trichloroéthane technique à l'état fondu à 92°C.

Le 1,1-bis(chlorophényl)-2,2,2-trichloroéthane à l'état liquide est ajouté à la phase aqueuse basique d'une opération précédente de déshydrochloration et on initie la déshydrochloration avec l'ajout de 5 kg de chlorure de diméthyl benzyl lauryl ammonium tout en maintenant en agitation intense pendant 4 h à 100°C.

Ensuite, on opère comme dans l'exemple 5, on obtient les mêmes résultats.

## Revendications

1. Procédé de déshydrochloration des 1,1-bis(R-phényl)-2,2,2-trichloroéthane, dans lesquels R représente un ou plusieurs halogènes, alkyls, groupes hydroxy (OH), groupes NO₂ ou alkoxy, à l'aide d'une solution aqueuse d'un hydroxyde alcalin caractérisé en ce qu'on opère en l'absence de solvant et en présence d'un agent de transfert de phase.

2. Procédé selon la revendication 1 caractérisé en ce que l'agent de transfert de phase est le chlorure de diméthyl benzyl lauryl ammonium.

## Claims

1. Process for the dehydrochlorination of 1,1-bis(R-phenyl)-2,2,2-trichloroethanes, in which R represents one or a number of halogens, alkyls, hydroxy (OH) groups or NO₂ or alkoxy groups, using an aqueous alkali metal hydroxide solution, characterized in that the reaction is carried out in the absence of solvent and in the presence of a phase transfer agent.

2. Process according to Claim 1, characterized in that the phase transfer agent is dimethylbenzyllaurylammonium chloride.

## Patentansprüche

1. Verfahren zur Dehydrochlorierung der 1,1-Bis(R-phenyl)-2,2,2-trichlorethane, wobei R ein oder mehrere Halogenatom(e), Alkyl-, Hydroxygruppen (OH), NO₂- oder Alkoxygruppen darstellt, mit Hilfe einer wäßrigen Lösung eines Alkalimetallhydroxids, dadurch gekennzeichnet, daß man ohne Lösemittel und in Gegenwart eines Phasenübertragungsmittels verfährt.

2. Verfahren nach Anspruch 1, dadurch gekennzeichnet, daß das Phasenübertragungsmittel Dimethylbenzyllaurylammoniumchlorid ist.
